# EUROPÄISCHE PATENTANMELDUNG

(11) **EP 2 000 175 A2**
(43) Veröffentlichungstag der Anmeldung: **10.12.2008**
(21) Anmeldenummer: 08103736.8
(22) Anmeldetag: 28.04.2008
(51) Int. Cl.: A61N 5/10

(54) **Steuervorrichtung und Verfahren zur Steuerung einer Strahlentherapievorrichtung sowie Strahlentherapievorrichtung**

(30) Priorität: 08.06.2007 DE 102007026516
(71) Anmelder: SIEMENS AKTIENGESELLSCHAFT, 80333 München (DE)
(72) Erfinder: Rietzel, Eike, 64289 Darmstadt (DE)

(57) **Zusammenfassung**

Die Erfindung betrifft eine Steuervorrichtung zur Steuerung einer Strahlentherapievorrichtung, mit der ein zu bestrahlendes Zielvolumen (35) bestrahlbar ist, umfassend einen Sensor (37), mit dem ein Druck im Inneren eines Körpers messbar ist, und eine Einheit (51) zur Ausgabe eines Steuersignals für eine Strahlunterbrechungseinheit (53) in Abhängigkeit von einem Druckzustand im Inneren des Körpers. Weiterhin betrifft die Erfindung eine Strahlentherapievorrichtung zur Bestrahlung eines zu bestrahlenden Zielvolumens (35) in einem Körper mit einer Strahlunterbrechungseinheit (55) und mit einer derartigen Steuervorrichtung. Weiterhin betrifft die Erfindung ein Verfahren zur Steuerung einer Strahlentherapievorrichtung, umfassend ein Messen eines Drucks im Inneren eines Körpers mithilfe eines Sensors (37), und ein Unterbrechen eines Bestrahlungsvorgangs in Abhängigkeit von einem Druckzustand im Inneren des Körpers.

## Beschreibung

Die Erfindung betrifft eine Steuervorrichtung und ein Verfahren zur Steuerung einer Strahlentherapievorrichtung sowie eine Strahlentherapievorrichtung.

Die Strahlentherapie gehört zu den etablierten Methoden insbesondere zur Behandlung von Tumorerkrankungen. Hierbei werden Strahlen auf ein zu bestrahlendes Zielvolumen eines Körpers gerichtet. Im Rahmen der Partikeltherapie werden beispielsweise Protonen, Kohlenstoffionen oder andere Ionen auf hohe Energien beschleunigt und auf das zu bestrahlenden Gewebe gerichtet. Es können aber auch andere Strahlenarten wie beispielsweise Elektronen- oder Röntgenstrahlen zur Bestrahlung verwendet werden. Die Interaktion des Behandlungsstrahls mit dem Gewebe führt zu einer Abtötung des bestrahlten Gewebes.

Um ein Zielvolumen möglichst genau zu bestrahlend, ist es wichtig, dass der Behandlungsstrahl seine Energie möglichst nur im Zielvolumen abgibt und umliegendes Gewebe geschont wird. Bewegungen des zu bestrahlenden Gewebes können jedoch dazu führen, dass das Zielvolumen nicht wie geplant vollständig getroffen wird. Ein Teil der Energie des Behandlungsstrahls kann durch die Bewegung im zu schonenden Gewebe abgegeben werden. Dies führt zu einer Qualitätsminderung der Bestrahlung und zu erhöhten Nebenwirkungen.

Seit einigen Jahren ist es möglich, über Bildgebung, die direkt vor der Bestrahlung durchgeführt wird, die interne Patientenanatomie zu erfassen. So kann zu Beginn einer Bestrahlungssitzung sichergestellt werden, dass das zu bestrahlende Volumen möglichst genau positioniert und von dem Behandlungsstrahl getroffen wird. Basierend auf der Bildgebung können die Patientenposition und/oder Bestrahlungsparameter auf die durch die Bildgebung ermittelte Situation angepasst werden (so genannte ART, "adaptive Strahlentherapie").

Auch während des Bestrahlungsvorgangs selbst kann eine Bewegung des zu bestrahlenden Zielvolumens auftreten. In jüngster Zeit gibt es Bestrebungen dahingehend, die Bewegungen während eines Bestrahlungsvorgangs zu detektieren und die Bestrahlung während des Bestrahlungsvorgangs entsprechend anzupassen.

In der US 2003/0136924 A1 ist eine Vorrichtung und ein Verfahren offenbart, bei der die Bewegung während eines Bestrahlungsvorgangs mithilfe von optischen Kameras und/oder Röntgensystemen überwacht wird und ein Ionenstrahl der Bewegung während des Bestrahlungsvorgangs angepasst wird.

Die Firma Calypso Medical Technologies Inc. bietet ein System mit Hochfrequenz-Markern an, die in eine Prostata implantiert werden können. Hierüber kann eine Abweichung des Zielvolumens von einer geplanten Bestrahlungsposition detektiert werden.

Es ist die Aufgabe der Erfindung, eine Steuervorrichtung und ein Verfahren zur Steuerung einer Strahlentherapievorrichtung anzugeben, mit dem eine Bestrahlung eines bewegten Zielvolumens auf einfache Weise sicher und nebenwirkungsarm durchgeführt werden kann. Weiterhin ist es die Aufgabe der Erfindung, eine Strahlentherapievorrichtung zu diesem Zweck bereitzustellen.

Die Aufgabe wird erfindungsgemäß gelöst durch eine Steuervorrichtung nach Anspruch 1, durch eine Strahlentherapievorrichtung nach Anspruch 9 sowie durch ein Verfahren nach Anspruch 11. Vorteilhafte Weiterbildungen finden sich in den Merkmalen der abhängigen Ansprüche.

Die Steuervorrichtung zur Steuerung einer Strahlentherapievorrichtung, mit der ein zu bestrahlendes Zielvolumen bestrahlbar ist, umfasst:
- einen Sensor, mit dem ein Druck im Inneren eines Körpers messbar ist,
- eine Einheit zur Ausgabe eines Steuersignals für eine Strahlunterbrechungseinheit in Abhängigkeit von einem Druckzustand im Inneren des Körpers.

Der Erfindung liegt die Erkenntnis zu Grunde, dass Druckschwankungen im Inneren eines Körpers auftreten können und dass diese Druckschwankungen oftmals zu einer Verschiebung der Lage des Zielvolumens führen können. Indem diese Druckschwankungen gemessen werden, kann ein Bestrahlungsvorgang verbessert gesteuert werden. In Abhängigkeit des gemessenen Drucks kann eine Unterbrechung der Bestrahlung erfolgen, so dass immer dann die Bestrahlung unterbrochen wird, wenn durch eine Druckschwankung eine Verschiebung der Position des Zielvolumens wahrscheinlich ist. Hierdurch ist es möglich, Bewegungen im Inneren des Körpers während der Bestrahlung zu berücksichtigen, ohne dass aufwändige Marker implantiert werden müssen, oder ohne dass eine zusätzliche Durchleuchtung des Körpers durchgeführt werden muss. Insgesamt lässt sich so erreichen, dass eine Lageänderung des zu bestrahlenden Zielvolumens - hervorgerufen durch Druckschwankungen im Inneren des Körpers -, nicht zu einer Fehlbestrahlung des Zielvolumens führt.

In vielen Fällen ist die Positionsänderung des Zielvolumens weitgehend reversibel, sobald sich der Druck normalisiert hat. Daher kann eine Bestrahlung wieder aufgenommen werden, wenn durch den Sensor eine Normalisierung des gemessenen Drucks detektiert wird.

In einer vorteilhaften Ausführungsform ist der Sensor zur Druckmessung in einem Hohlorgan des Körpers ausgebildet. Hierdurch können Druckschwankungen in Organen gemessen werden, die häufig Druckschwankungen unterliegen, nämlich in Hohlorganen. Die Druckmessung muss jedoch nicht zwangsläufig in einem Hohlorgan erfolgen; es kann beispielsweise auch der Druck innerhalb der Bauchdecke oder innerhalb anderer Gewebe/Organe gemessen werden, die nicht als Hohlorgan ausgebildet sind.

In einer vorteilhaften Ausführungsvariante ist der Sensor zur Druckmessung im Darm ausgebildet. Hierdurch können Druckschwankungen gemessen werden, die insbesondere durch intestinale Gase bzw. durch Bewegung von intestinalen Gasmassen erzeugt werden. Auf diese Weise können viele Bestrahlungsvorgänge, die im Bereich des Darms durchgeführt werden, verbessert werden. Hierzu gehören beispielsweise Bestrahlungen der Prostata, Bestrahlungen im Unterleib und Bestrahlungen im Abdomen. Die Druckmessung kann jedoch auch in anderen Hohlorganen, wie beispielsweise der Blase, der Lunge, dem Magen oder anderen stattfinden.

In einer vorteilhaften Ausführungsvariante ist der Sensor derart ausgebildet, dass er rektal in den Darm einführbar ist. Dieser einfache Zugangsweg erlaubt ein problemloses Anlegen des Sensors an einen Patienten.

In einer Ausführungsvariante ist der Sensor zur Druckmessung eines Gasdrucks ausgebildet. Auf diese Weise können z.B. Druckschwankungen im Darm, die durch intestinale Gasmassen hervorgerufen werden, detektiert werden.

Der Sensor kann in vorteilhafter Weise an einem System mit einem Ventilmechanismus angebracht sein, das zum Ablassen des Überdrucks ist. Beispielsweise kann über den Ventilmechanismus ein im Darm entstehender Überdruck rektal abgelassen werden. Auf diese Weise normalisieren sich die Druckverhältnisse im Darm schneller, so dass eine Fortführung des Bestrahlungsvorgangs schneller wieder aufgenommen werden kann. Vorteilhafterweise bleibt das Ventil solange geöffnet, bis der gesamte Überdruck im Darm abgelassen ist.

In vorteilhafter Weise kann der Sensor an einem aufblasbaren Ballon angeordnet sein. Ein aufblasbarer rektaler Ballon z.B. unterstützt zusätzlich die Immobilisation von Organen, insbesondere der Prostata.

Mit Vorteil umfasst die Steuervorrichtung eine Auswertungseinheit, mit der ein vom Sensor erzeugtes Sensorsignal ausgewertet wird. Die Ausgabe des Steuersignals für eine Strahlunterbrechungseinheit erfolgt in diesem Falle in Abhängigkeit von einer Auswertung der Auswertungseinheit. Mit der Auswertungseinheit können auf einfache Weise komplexe Algorithmen zur Auswertung implementiert und angepasst werden.

In einer Ausführungsform kann beispielsweise immer dann ein temporärer Strahlabbruch induziert werden, wenn von der Auswertungseinheit eine Auswertung des Sensorsignals von einem Toleranzbereich oder eine Überschreitung eines Schwellenwertes festgestellt wird.

Die Strahlentherapievorrichtung zur Bestrahlung eines zu bestrahlenden Zielvolumens in einem Körper umfasst eine Strahlunterbrechungseinheit und eine Steuervorrichtung nach einem der Ansprüche 1 bis 9. In einer Ausführungsvariante ist die Strahlentherapievorrichtung als Partikeltherapieanlage ausgebildet. Es ist aber auch möglich, die Strahlentherapievorrichtung als herkömmliches Strahlentherapiegerät, das beispielsweise mit einem LINAC zur Erzeugung von hochenergetischen Röntgenstrahlen oder Elektronenstrahlen auszubilden.

Das Verfahren zur Steuerung einer Strahlentherapievorrichtung umfasst folgende Schritte:
- Messen eines Drucks in einem Körper mithilfe eines Sensors, und
- Unterbrechen eines Bestrahlungsvorgangs in Abhängigkeit von einem Druckzustand im Inneren des Körpers.

Mit dem Verfahren lässt sich analog zur Vorrichtung erreichen, dass eine Lageänderung des zu bestrahlenden Zielvolumens - hervorgerufen durch Druckschwankungen im Inneren des Körpers -, nicht zu einer Fehlbestrahlung des Zielvolumens führt.

Der Druck kann dabei in einem Hohlorgan des Körpers, insbesondere in einem Darm gemessen werden, wobei der Sensor insbesondere rektal in den Darm eingeführt wird. Als Druck im Inneren des Körpers kann beispielsweise ein Gasdruck gemessen werden.

Vorteilhafterweise wird ein von dem Sensor erzeugtes Sensorsignal ausgewertet, wobei das Unterbrechen des Bestrahlungsvorgangs in Abhängigkeit von einer Auswertung des Sensorsignals erfolgt. Beispielsweise wird der Bestrahlungsvorgang immer dann temporär unterbrochen, wenn durch die Auswertung eine Abweichung des Sensorsignals von einem Toleranzbereich oder ein Überschreiten eines Schwellenwertes festgestellt wird.

Ausführungsformen der vorliegenden Erfindung sowie vorteilhafte Ausgestaltungen gemäß den Merkmalen der unabhängigen Ansprüche werden anhand der folgenden Zeichnung näher erläutert, ohne jedoch darauf beschränkt zu sein.

Es zeigen:
- Figur 1: einen schematischen Überblick über eine Partikelthe-rapieanlage,
- Figur 2: einen schematischen Sagittalschnitt durch einen menschlichen Körper,
- Figur 3: eine schematische Darstellung eines Bestrahlungsvorgangs, und
- Figur 4: einen schematischen Überblick über die einzelnen Verfahrensschritte.

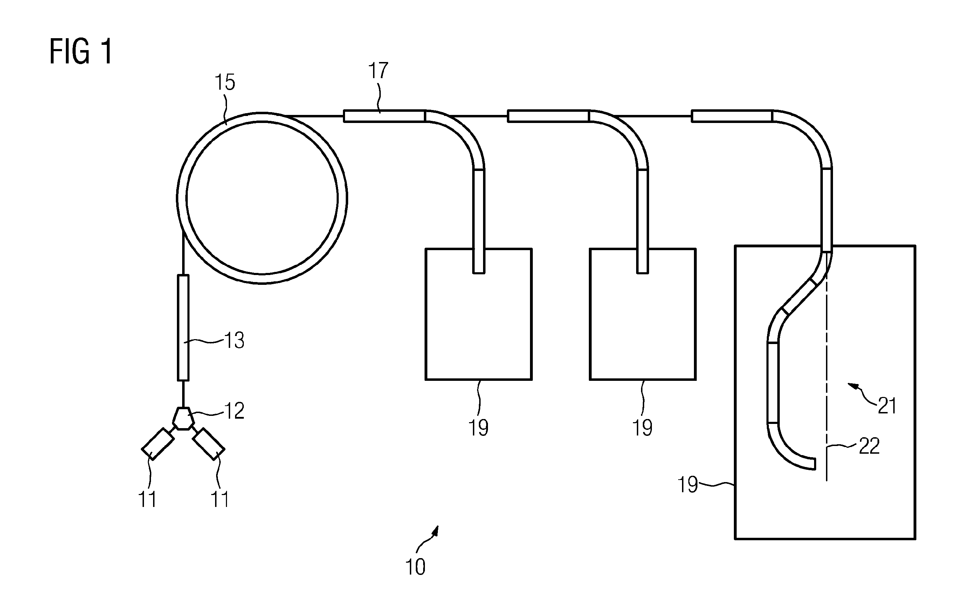

Figur 1 zeigt einen schematischen Überblick über den Aufbau einer Partikeltherapieanlage 10. In einer Partikeltherapieanlage 10 erfolgt eine Bestrahlung eines Körpers, insbesondere eines tumorerkrankten Gewebes, mit einem Partikelstrahl.

Als Partikel werden vornehmlich Ionen wie beispielsweise Protonen, Pionen, Heliumionen, Kohlenstoffionen oder andere Ionensorten eingesetzt. Üblicherweise werden derartige Partikel in einer Partikelquelle 11 erzeugt. Wenn, wie in Figur 1 dargestellt, zwei Partikelquellen 11 vorhanden sind, die zwei verschiedene Ionensorten erzeugen, kann zwischen diesen beiden Ionensorten innerhalb eines kurzen Zeitintervalls umgeschaltet werden. Dazu wird beispielsweise ein Schaltmagnet 12 verwendet, der zwischen den Ionenquellen 11 einerseits und einem Vorbeschleuniger 13 andererseits angeordnet ist. Z.B. kann hierdurch die Partikeltherapieanlage 10 mit Protonen und mit Kohlenstoffionen gleichzeitig betrieben werden.

Die von der oder einer der Ionenquellen 11 erzeugten und gegebenenfalls mit dem Schaltmagneten 12 ausgewählten Ionen werden in dem Vorbeschleuniger 13 auf ein erstes Energieniveau beschleunigt. Der Vorbeschleuniger 13 ist beispielsweise ein Linearbeschleuniger (LINAC für engl.: "LINear ACcelerator"). Anschließend werden die Partikel in einen Beschleuniger 15, beispielsweise ein Synchrotron oder Zyklotron, eingespeist. In dem Beschleuniger 15 werden sie auf hohe Energien, wie sie zur Bestrahlung nötig sind, beschleunigt. Nachdem die Partikel den Beschleuniger 15 verlassen, führt ein Hochenergiestrahl-Transportsystem 17 den Partikelstrahl zu einem oder mehreren Bestrahlungsräumen 19. In einem Bestrahlungsraum 19 werden die beschleunigten Partikel auf einen zu bestrahlenden Körper gerichtet. Je nach Ausgestaltung erfolgt dies von einer festen Richtung (in so genannten "fixed beam"-Räumen) aus oder aber über eine um eine Achse 22 bewegliche rotierbare Gantry 21 von verschiedenen Richtungen aus.

Der anhand der Figur 1 dargestellte Grundaufbau einer Partikeltherapieanlage 10 ist typisch für viele Partikeltherapieanlagen, kann aber auch hiervon abweichen. Die nachfolgend beschriebenen Ausführungsbeispiele sind sowohl in Zusammenhang mit der anhand der Figur 1 dargestellten Partikeltherapieanlage als auch mit anderen Partikeltherapieanlagen oder Strahlentherapievorrichtungen bzw. -geräten einsetzbar.

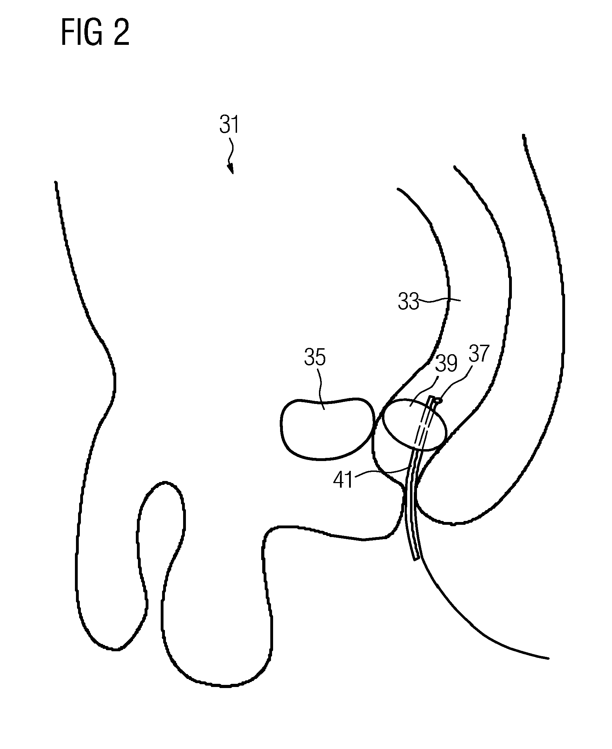

Figur 2 zeigt einen schematischen Sagittalschnitt 31 durch den Unterleib eines männlichen Körpers. Schematisch angedeutet sind lediglich die äußeren Konturen sowie die Konturen der Organe, die für die nachfolgenden Erläuterungen notwendig sind. Im Unterleib nahe des Rektums 33 befindet sich die Prostata 35, ein Organ, dessen Tumore häufig im Rahmen einer Strahlentherapie behandelt werden. Es hat sich gezeigt, dass während einer Bestrahlung die Prostata 35 Bewegungen innerhalb des Körpers, d.h. interne Bewegungen, durchführen kann. Diese sind überwiegend durch eine Gasentwicklung und/oder Verschiebung von Gasmassen im benachbarten Darm bzw. im benachbarten Rektum 33 verursacht.

Im Rektum 33 ist rektal ein Sensor 37 eingeführt, mit dem der Druck im Inneren des Darmes gemessen werden kann. Der Sensor 37 ist in diesem speziellen Ausführungsbeispiel an einem so genannten rektalen Ballon 39 angeordnet, der zur Immobilisation der Prostata 35 eingesetzt werden kann. Der rektale Ballon 39 wird hierfür in einem zusammengefalteten Zustand ins Rektum 33 eingeführt. Durch Aufblasen des rektalen Ballons 33 lässt sich bis zu einem gewissen Grad eine mechanische Fixierung der Prostata 35 erreichen.

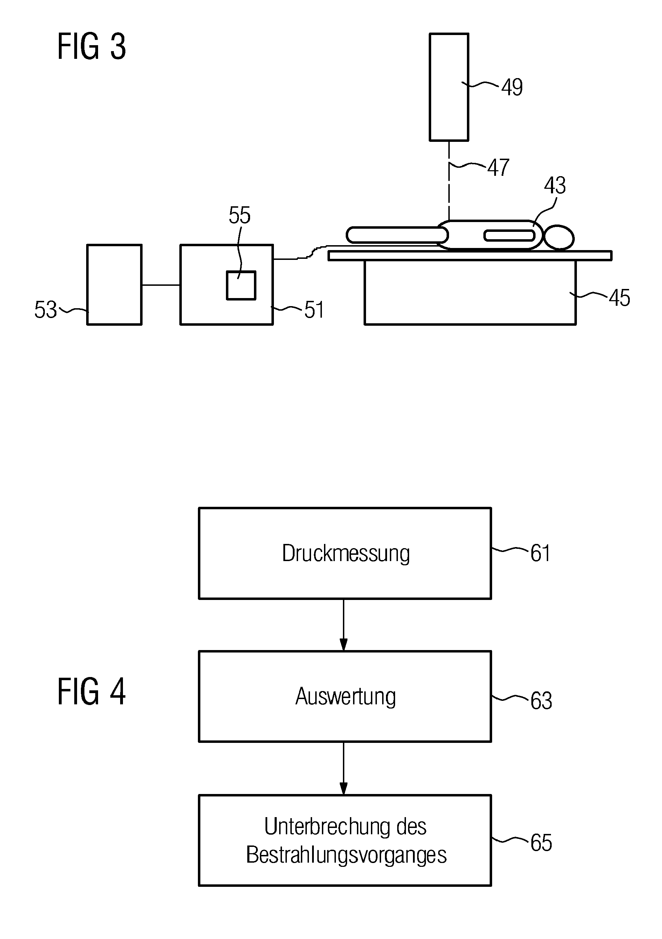

Trotz der Immobilisation ist es möglich, dass ein durch Gasmassen verursachter Druckanstieg im Rektum 33 bzw. im Darm die Lage der Prostata 35 verschieben kann. Durch den Sensor 37 können die Druckschwankungen detektiert werden, so dass hierauf basierend der Bestrahlungsvorgang unterbrochen werden kann, wie später anhand von Figur 3 ausführlicher erläutert wird.

Der hier gezeigte rektale Ballon 39 verfügt zudem über einen Ventilmechanismus 41, so dass intestinale Gasmassen bei einem Druckanstieg entweichen können. Hierzu öffnet ein Ventil, sobald der intestinale Druck einen Schwellenwert erreicht hat und bleibt vorzugsweise solange offen, bis die Gasmassen soweit entwichen sind, dass der rektale bzw. intestinale Überdruck wieder normalisiert ist. Auf diese Weise kann ein im Darm entstehender Überdruck schnell ausgeglichen werden. In vielen Fällen bewirkt dies ein Zurückkehren des zu bestrahlenden Organs an seine ursprüngliche Position, so dass eine unterbrochene Bestrahlung wieder aufgenommen werden kann.

Figur 3 zeigt schematisch einen Patienten 43, der auf einer Patientenliege 45 in einem Behandlungsraum derart positioniert ist, dass der zu bestrahlende Bereich im Unterleib durch einen Partikelstrahl 47, der von einem Strahlauslass 49 zu dem Patienten gerichtet ist, getroffen wird. Durch den in den Darm des Patienten eingeführten Sensor wird ein Sensorsignal aufgezeichnet, das die Druckverhältnisse im Darm kennzeichnet.

Das Sensorsignal wird daraufhin zu einer Therapiesteuervorrichtung 51 geleitet. In einem einfachen Fall erzeugt die Therapiesteuervorrichtung 51 die Ausgabe eines Steuersignals für eine Strahlunterbrechungseinheit 53, sobald der gemessene Druck einen kritischen Schwellenwert übersteigt. Alternativ kann die Ausgabe des Steuersignals für die Strahlunterbrechungseinheit 53 stets dann erfolgen, wenn der gemessene Druck außerhalb eines beispielsweise vorgegebenen Toleranzbereichs liegt.

Die Unterbrechung des Bestrahlungsvorgangs kann beispielsweise derart gesteuert werden, dass der Bestrahlungsvorgang unterbrochen wird, solange das Steuersignal für die Strahlunterbrechungseinheit 53 anliegt.

In einer komplexeren Ausführungsvariante kann das Sensorsignal zu einer Auswertungseinheit 55 geführt werden, die beispielsweise in der Therapiesteuervorrichtung 51 implementiert ist und in der komplexere Algorithmen zur Verarbeitung und zur Auswertung des Steuersignals auf einfache Weise implementiert und angepasst werden können.

Die einzelnen Einheiten, d.h. die Strahlunterbrechungseinheit 53, die Einheit zur Ausgabe eines Steuersignals für die Strahlunterbrechungseinheit bzw. die Therapiesteuervorrichtung 51 sowie die Auswertungseinheit 55 können auf verschiedene Art und Weise in einer Partikeltherapieanlage implementiert werden, beispielsweise als getrennte Einheiten oder zusammen in einer einzigen Rechnereinheit.

Figur 4 zeigt eine schematische Übersicht über die Verfahrensschritte.

In einem ersten Schritt 61 erfolgt die Messung des Drucks im Inneren eines Körpers, insbesondere im Inneren des Darmes. In einem zweiten optionalen Schritt 63 erfolgt die Auswertung eines Sensorsignals, das den Druck im Inneren des Darmes charakterisiert, beispielsweise durch eine Auswertungseinheit. In einem dritten Schritt 65 erfolgt die Unterbrechung des Bestrahlungsvorgangs in Abhängigkeit des Drucks im Inneren des Darmes, beispielsweise mithilfe eines Steuersignals für eine Strahlunterbrechungseinheit.

## Patentansprüche

1. Steuervorrichtung zur Steuerung einer Strahlentherapievorrichtung, mit der ein zu bestrahlendes Zielvolumen (35) bestrahlbar ist, umfassend:
- einen Sensor (37), mit dem ein Druck im Inneren eines Körpers messbar ist,
- eine Einheit (51) zur Ausgabe eines Steuersignals für eine Strahlunterbrechungseinheit (53) in Abhängigkeit von einem Druckzustand im Inneren des Körpers.

2. Steuervorrichtung nach Anspruch 1, wobei der Sensor (37) zur Druckmessung in einem Hohlorgan des Körpers ausgebildet ist.

3. Steuervorrichtung nach Anspruch 2, wobei der Sensor (37) zur Druckmessung im Darm (33) ausgebildet ist.

4. Steuervorrichtung nach Anspruch 3, wobei der Sensor (37) rektal in den Darm (33) einführbar ist.

5. Steuervorrichtung nach einem der Ansprüche 1 bis 4, wobei der Sensor (37) zur Druckmessung eines Gasdrucks ausgebildet ist.

6. Steuervorrichtung nach einem der Ansprüche 1 bis 5, wobei der Sensor (37) an einem Ventilmechanismus (41) zum Ablassen eines Überdrucks angeordnet ist.

7. Steuervorrichtung nach einem der Ansprüche 1 bis 6, wobei der Sensor (37) an einem aufblasbaren Ballon (39) angeordnet ist.

8. Steuervorrichtung nach einem der Ansprüche 1 bis 7, wobei ein von dem Sensor (37) erzeugtes Sensorsignal von einer Auswertungseinheit (55) auswertbar ist, so dass die Ausgabe des Steuersignals für eine Strahlunterbrechungseinheit (53) in Abhängigkeit von einer Auswertung der Auswertungseinheit (55) erfolgt.

9. Steuervorrichtung nach einem der Ansprüche 1 bis 8, wobei durch das Steuersignal für eine Strahlunterbrechungseinheit (53) immer dann ein temporärer Strahlabbruch induzierbar ist, wenn eine Abweichung des Sensorsignals von einem Toleranzbereich oder eine Überschreitung eines Schwellenwertes festgestellt wird.

10. Strahlentherapievorrichtung zur Bestrahlung eines zu bestrahlenden Zielvolumens (35) in einem Körper mit einer Strahlunterbrechungseinheit (55) und mit einer Steuervorrichtung nach einem der Ansprüche 1 bis 9.

11. Strahlentherapievorrichtung nach Anspruch 10, die als Partikeltherapieanlage (10) ausgebildet ist.

12. Verfahren zur Steuerung einer Strahlentherapievorrichtung, umfassend folgende Schritte:
- Messen eines Drucks im Inneren eines Körpers mithilfe eines Sensors (37),
- Unterbrechen eines Bestrahlungsvorgangs in Abhängigkeit von einem Druckzustand im Inneren des Körpers.

13. Verfahren nach Anspruch 12, wobei Druck in einem Hohlorgan des Körpers gemessen wird.

14. Verfahren nach Anspruch 13, wobei der Druck in einem Darm (33) gemessen wird.

15. Verfahren nach einem der Ansprüche 12 bis 14, wobei mit dem Sensor (37) ein Gasdruck im Inneren eines Körpers gemessen wird.

16. Verfahren nach einem der Ansprüche 12 bis 15, wobei ein von dem Sensor erzeugtes Sensorsignal ausgewertet wird und das Unterbrechen des Bestrahlungsvorgangs in Abhängigkeit von einer Auswertung des Sensorsignals erfolgt.

17. Verfahren nach einem der Ansprüche 12 bis 16, wobei der Bestrahlungsvorgang immer dann temporärer unterbrochen wird, wenn eine Abweichung des Sensorsignals von einem Toleranzbereich oder eine Überschreitung eines Schwellenwertes festgestellt wird.
